(19) Europäisches Patentamt
European Patent Office
Office européen des brevets

(11) Publication number: **0 206 361 B1**

(12) # EUROPEAN PATENT SPECIFICATION

(45) Date of publication of the patent specification: **26.04.89**

(51) Int. Cl.[4]: **C07C 103/84**

(21) Application number: **86110216.8**

(22) Date of filing: **12.08.82**

(60) Publication number of the earlier application in accordance with Art. 76 EPC: **0076030**

(54) **Methyl benzoylamino-2-hydroxyacetates.**

(30) Priority: **30.09.81 GB 8129521**

(43) Date of publication of application: **30.12.86 Bulletin 86/52**

(45) Publication of the grant of the patent: **26.04.89 Bulletin 89/17**

(84) Designated Contracting States: **AT BE CH DE FR GB IT LI NL SE**

(56) References cited:
**EP-A- 0 059 536**
**EP-A- 0 061 836**

**TETRAHEDRON, vol. 33, no. 8, 1977, pages 881-883, Pergamon Press, GB; Z.BERNSTEIN et al.: "Synthesis of N-Substituted azirdine-2-Carboxylates**

(73) Proprietor: **IMPERIAL CHEMICAL INDUSTRIES PLC, Imperial Chemical House Millbank, London SW1P 3JF(GB)**

(72) Inventor: **Kay, Ian Trevor, "Portelet" Kiln Ride Extension, Wokingham Berkshire(GB)**
Inventor: **Noon, Robert Andrew, 16 Allenby Road, Maidenhead Berkshire(GB)**

(74) Representative: **Alner, Henry Giveen Hamilton et al, Imperial Chemical Industries PLC Legal Department: Patents P.O. Box 6 Bessemer Road, Welwyn Garden City Herts, AL7 1HD(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 206 361 B1

ACTORUM AG

## Description

This invention relates to methyl benzoylamino-2-hydroxyacetates and to their preparation.

According to the invention there are provided compounds of the general formula (VI):

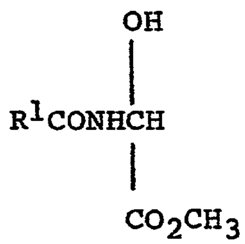

(VI)

wherein $R^1$ is:

(a) a phenyl group substituted by one or more halogen atoms other than 3,5-dichlorophenyl, cyano groups, $C_{1-6}$ alkoxy groups, methylene- or ethylenedioxy groups, $C_{1-6}$ alkyl groups or $C_{1-6}$ haloalkyl groups (e.g. trifluoromethyl groups); or

(b) a furyl, thienyl or pyridyl group optionally substituted by one or more $C_{1-6}$ alkyl groups, $C_{1-6}$ haloalkyl groups or halogen atoms, but excluding methyl-(3-methoxybenzoylamino (hydroxyl)) acetate.

In the foregoing definition, the term "halogen" is intended to include fluorine, chlorine, bromine and iodine. Particular examples of the terms alkyl, alkoxy and haloalkyl are methyl, ethyl, propyl, isopropyl, methoxy, ethoxy and trifluoromethyl.

The compounds of the invention are useful as intermediates in the preparation of the amide described and claimed in our European Patent Application 0 076 030.

The present invention is concerned particularly with compounds in which $R^1$ is phenyl substituted at one or more of the 3-, 4- or 5-positions with one or more halogens other than 3,5-dichlorophenyl or $C_{1-6}$ alkyl, $C_{1-6}$ alkoxy, trifluoromethyl or methylene or ethylene-dioxy groups.

Thus, $R^1$ can be, for instance, phenyl substituted at the either 3- or 4-position, or both, with chlorine, fluorine, bromine, methyl, methoxy or methylenedioxy (3,4-only). Examples of suitable values for $R^1$ are: 3,4-$OCH_2O$·$C_6H_3$, 4-Cl·$C_6H_4$, 4-$CH_3$·$C_6H_4$, 4-Br·$C_6H_4$, 4-$CH_3O$·$C_6H_4$, 3-F,4-$CH_3$·$C_6H_3$ or 4-Cl-3,F-$C_6H_3$.

Particular examples of values for $R^1$ of compounds of the invention are listed below in Table I.

Table I

| Compound Number | $R^1$ |
|---|---|
| 1 | 3-Cl . $C_6H_4$ |
| 2 | 3-F . $C_6H_4$ |
| 3 | 3-Br . $C_6H_4$ |
| 4 | 3,5-$(CH_3)_2C_6H_3$ |
| 5 | 3,F-4-$CH_3O$ . $C_6H_3$ |
| 6 | 3-$CF_3$ . $C_6H_4$ |
| 7 | 3-$CH_3$ . $C_6H_4$ |
| 8 | 2-furyl |
| 9 | 3,4-$Cl_2C_6H_3$ |
| 10 | 5-bromo-2-furyl |
| 11 | 3-I . $C_6H_4$ |
| 12 | 4-F . $C_6H_4$ |
| 13 | 3,4-$(CH_3)_2$ . $C_6H_3$ |
| 14 | 2-bromo-5-furyl |
| 15 | 2-$CH_3$-5-thienyl |
| 16 | 3,5-$Br_2$ . $C_6H_3$ |
| 17 | 3,5-$F_2$ . $C_6H_3$ |
| 18 | 3,4-$OCH_2O$ . $C_6H_3$ |
| 19 | 4-Cl . $C_6H_4$ |
| 20 | 4-$CH_3$ . $C_6H_4$ |
| 21 | 4-Br . $C_6H_4$ |
| 22 | 4-$CH_3O$ . $C_6H_4$ |
| 23 | 3-F,4-$CH_3$ . $C_6H_3$ |
| 24 | 4-Cl-3,F-$C_6H_3$ |
| 25 | 4-Br-3,F . $C_6H_3$ |
| 26 | 4,Me-3,Br . $C_6H_3$ |
| 27 | 4,Cl-3,Br . $C_6H_3$ |

In the foregoing Table Compound Nos 18, 19, 20, 21, 22, 23 and 24, are of particular interest because they are intermediates for especially useful fungicidal compounds of European Application 0 076 030.

The structural formula given above is believed to be the one which best represents the structure of the compounds of the invention. For some compounds within the scope of the formula (VI) it may be possible in principle for tautomeric forms of the compounds to exist, in which a hydrogen atom is transposed from the position in which it is shown in formula (VI) to another part of the molecule, and the chemical bonds between the atoms of the molecule are consequently rearranged. The structural formula (VI) is intended to represent and include such tautomeric forms, insofar as they may exist.

It will be noted that the formula (VI) includes an asymmetric carbon atom (the one to which the hydroxyl group depicted is attached) and the molecule is therefore capable in principle of existing in two optically isomeric forms (D and L forms). The present invention includes the separate D and L forms of the compounds as well as mixtures of the D and L forms in all proportions. As normally prepared by chemical synthesis the compounds are obtained as mixtures of equal proportions of the D and L forms (i.e. racemic mixtures). Methods of separating racemic mixtures into the D and L forms are well known in the art.

The structural formula (VI) is also intended to include any physically distinguishable modifications of the compounds which may arise, for example, from different ways in which the molecules are arranged in a crystal lattice, or from the inability of parts of the molecules to rotate freely in relation to other parts, or from geometrical isomerism, or from intra-molecular or inter-molecular hydrogen bonding, or otherwise.

The invention further provides a process for preparing compounds of formula (VI) above, which comprises bringing into reaction an amide of the formula $R^1CONH_2$, where $R^1$ is as defined above, with methyl glyoxylate. This process can be represented as follows:

Scheme

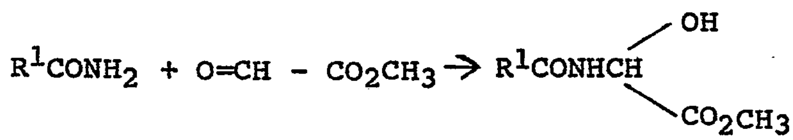

(VI)

This process is conveniently carried out in a solvent and may be accelerated by heating. Examples of solvents include liquid hydrocarbons, for example toluene and the xylenes. The reaction may be carried out at temperatures in the range from 50° C to 120C and preferably above 100°C. The hydroxy acetate product (VI) may be isolated by removal of the solvent. The hydroxy acetate (VI) can be converted into compounds of general formulae (VIII) and (IV) as follows with reference to the following scheme:

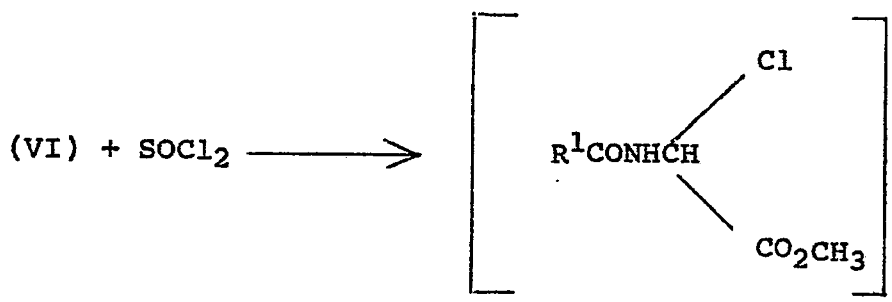

(VII)

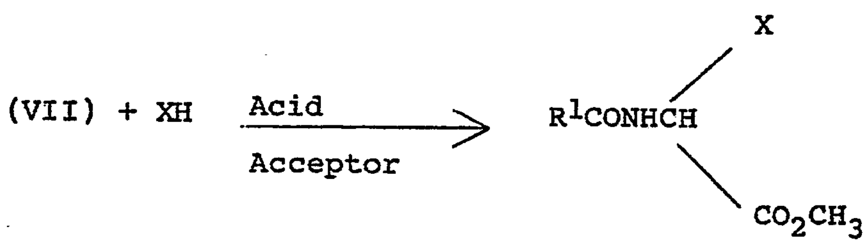


(VIII)

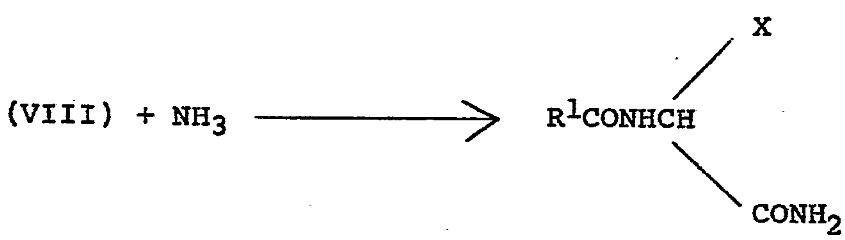

Thus, the hydroxy acetate can be converted to the unstable chloro-compound (VII) by treatment with thionyl chloride. Conveniently the reaction is carried out in an excess of thionyl chloride as solvent. The reaction proceeds at moderate temperatures (for example, temperatures in the range from 15°C–50°C). The chloro compound (VII) may be isolated by removal of the excess of thionyl chloride under reduced pressure. The chloro compound (VII) is then reacted with a 5-membered heteroaromatic compound XH (where X is 1-pyrazolyl, 1-(1,2,4-triazolyl) or 1-tetrazolyl, each optionally substituted by one or more $C_{1-6}$ alkyl groups) in the presence of an acid acceptor. Examples of acid acceptors include tertiary

amines, for example triethylamine, pyridine, and dimethylaniline. Conveniently the reaction may be performed using an excess of pyridine as both solvent and acid acceptor. The reaction proceeds at moderate temperatures (i.e. temperatures in the range 15°C–10°C).

The ester product (VIII) from the foregoing reaction is then reacted with ammonia to give the amide intermediate (IV); the reaction with ammonia is conveniently carried out in a solvent, for example a lower alkanol (e.g. methanol or ethanol) or an ether (e.g. diethyl ether or tetrahydrofuran). The reaction may be carried out by saturating the solution of the ester (VIII) with ammonia at a lowered temperature (e.g. –50°C to –10°C) and subsequently allowing the mixture to warm to room temperature. The amide (IV) so obtaine may then be converted if desired to the corresponding nitrile b dehydration, for instance by treatment of a solution of (IV) in pyridine with trifluoroacetic anhdyride. This reaction is generally exothermic and is preferably carried out below 0°C, for example at a temperature in the range from –20°C to –10°C. The nitrile thus obtained may be isolated from the reaction mixture by conventional methods.

The process of the invention is illustrated by the following Example.

EXAMPLE

Methyl 2-(3,5-dimethylbenzoylamino)-2-hydroxy acetate was prepared by refluxing equimolar proportions of 3,5-dimethylbenzamide and methyl glyoxylate in toluene and then removing the solvent under reduced pressure. The hydroxy acetate was obtained in a yield of 3 grams.

**Claims for the Contracting States BE, CH, DE, FR, GB, IT, LI, NL, SE:**

1. Compounds of the general formula (VI) :

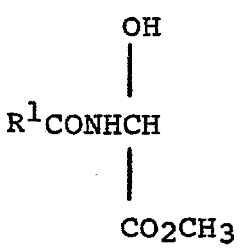

(VI)

wherein $R^1$ is:

(a) a phenyl group optionally substituted by one or more halogen atoms other than 3,5-dichlorophenyl, cyano groups, $C_{1-6}$ alkoxy group, methylene- or ethylenedioxy groups, $C_{1-6}$ alkyl groups or $C_{1-6}$ haloalkyl groups (e.g. trifluoromethyl groups); or

(b) a furyl, thienyl or pyridyl group optionally substituted by one or more $C_{1-6}$ alkyl groups, $C_{1-6}$ haloalkyl groups or halogen atoms, but excluding methyl-(3-methoxybenzoylamino(hydroxy)) acetate.

2. Compounds according to claim 1, wherein $R^1$ is phenyl substituted at one or more of the 3-, 4- or 5-positions with one or more halogens or $C_{1-6}$ alkyl, $C_{1-6}$ alkoxy, trifluoromethyl or methylene- or ethylenedioxy groups.

3. Compounds according to claim 1 or claim 2, wherein $R^1$ is phenyl substituted at the either 3- or 4-position, or both, with chlorine, fluorine, bromine, methyl, methoxy or methylenedioxy (3,4-only).

4. A compound of the formula (VI) wherein:
$R^1$ is 3,4-$OCH_2O \cdot C_6H_3$, 4-$Cl \cdot C_6H_4$, 4-$CH_3 \cdot C_6H_4$, 4-$Br \cdot C_6H_4$, 4-$CH_3O \cdot C_6H_4$, 3-F,4-$CH_3 \cdot C_6H_3$ or 4-Cl-3,F-$C_6H_3$.

5. A compound as defined in any of the preceding claims, which is in the form of a stereoisomer.

6. A process for preparing a compound claimed in claim 1, which comprises bringing into reaction an amide of the formula $R^1CONH_2$ where $R^1$ is as defined in claim 1 with methyl glyoxylate.

7. A process according to claim 6, carried out in a liquid hydrocarbon solvent and at a temperature in the range of 50° to 120°C.

8. A process according to claim 7, in which the solvent is selected from toluene and xylenes.

9. A process according to claim 8, carried out at a temperature above 100°C.

**Claims for the Contracting State AT:**

1. A process for preparing a compound of the general formula (VI):

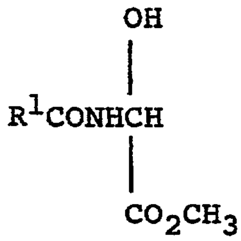

(I)

wherein $R^1$ is:

(a) a phenyl group optionally substituted by one or more halogen atoms other than 3,5-dichlorohenyl, cyano groups, $C_{1-6}$ alkoxy group, methylene- or ethylenedioxy groups, $C_{1-6}$ alkyl groups or $C_{1-6}$ haloalkyl groups (e.g. trifluoromethyl groups); or

(b) a furyl, thienyl or pyridyl group optionally substituted by one or more $C_{1-6}$ alkyl groups, $C_{1-6}$ haloalkyl groups or halogen atoms, but excluding methyl-(3-methoxybenzoylamino(hydroxy)) acetate, which comprises bringing into reaction an amide of the formula $R^1CONH_2$ where $R^1$ is as definded above with methyl glyoxylate.

2. A process according to claim 1, carried out in a liquid hydrocarbon solvent and at a temperature in the range of 50°C to 120°C.

3. A process according to claim 2, in which the solvent is selected from toluene and xylenes.

4. A process according to claim 3, carried out at a temperature above 100°C.

**Patentansprüche für die Vertragsstaaten BE, CH, DE, FR, GB, IT, LI, NL, SE:**

1. Verbindungen der allgemeinen Formel (VI)

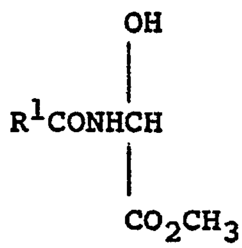

(VI)

worin $R^1$ für folgendes steht:

(a) eine Phenylgruppe, die ggf. durch ein oder mehrere Halogenatome, Cyanogruppen, $C_{1-6}$-Alkoxygruppen, Methylen- oder Ethylen-dioxygruppenen, $C_{1-6}$-Alkylgruppen oder $C_{1-6}$-Halogenoalkylgruppen (z.B. Trifluoromethylgruppen) substituiert ist;
oder

(b) eine Furyl-, Thienyl- oder Pyridylgruppe, die ggf. durch ein oder mehrere $C_{1-6}$-Alkylgruppen, $C_{1-6}$-Halogenoalkylgruppen oder Halogenatome substituiert ist,
wobei jedoch der (3-Methoxybenzoylamino(hydroxy))-essigsäuremethylester ausgeschlossen ist.

2. Verbindungen nach Anspruch 1, worin $R^1$ für Phenyl steht, bei dem ein oder mehrere der 3-, 4- und 5-Stellungen mit einem oder mehreren Halogenatomen oder $C_{1-6}$-Alkyl-, $C_{1-6}$-Alkoxy-, Trifluoromethyl- oder Methylen- oder Ethylen-dioxygruppen substituiert sind.

3. Verbindungen nach Anspruch 1 oder 2, worin $R^1$ für Phenyl steht, das in der 3- und/oder 4-Stellung mit Chlor, Fluor, Brom, Methyl, Methoxy oder Methylendioxy (nur 3, 4) substituiert ist.

4. Verbindung der Formel (VI), worin $R^1$ für 3,4-$OCH_2O \cdot C_6H_3$, 4-$Cl \cdot C_6H_4$, 4-$CH_3 \cdot C_6H_4$, 4-$Br \cdot C_6H_4$, 4-$CH_3O \cdot C_6H_4$, 3-F,4-$CH_3 \cdot C_6H_3$ oder 4-Cl-3, F-$C_6H_3$ steht.

5. Verbindungen nach einem der vorhergehenden Ansprüche, welche in der Form eines Stereoisomers vorliegen.

6. Verfahren zur Herstellung einer Verbindung nach Anspruch 1, bei welchem ein Amid der Formel $R^1CONH_2$, worin $R^1$ die in Anspruch 1 angegebene Definition besitzt, mit Methylglyoxylat in Reaktion gebracht wird.

7. Verfahren nach Anspruch 6, welches in einem flüssigen Kohlenwasserstofflösungsmittel und bei einer Temperatur im Bereich von 50 bis 120°C ausgeführt wird.

8. Verfahren nach Anspruch 7, bei welchem das Lösungsmittel aus Toluol und Xylolen ausgewählt wird.

9. Verfahren nach Anspruch 8, welches bei einer Temperatur über 100°C ausgeführt wird.

**Patentansprüche für den Vertragstaat AT:**

1. Verfahren zur Herstellung einer Verbindung der allgemeinen Formel (VI)

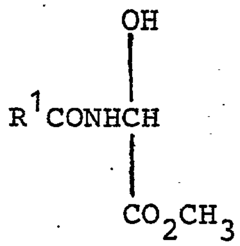

(VI)

worin $R^1$ für folgendes steht:
a) eine Phenylgruppe, die durch ein oder mehrere Halogenatome, wobei 3,5-Dichlorophenyl ausgenommen ist, Cyanogruppen, $C_{1-6}$-Alkoxygruppen, Methylen- oder Ethylen-Dioxygruppen, $C_{1-6}$-Alkylgruppen oder $C_{1-6}$-Halogenoalkylgruppen (z.B. Trifluoromethylgruppen) subsituiert ist;
oder
(b) eine Furyl-, Thienyl- oder Pyridylgruppe, die ggf. durch ein oder mehrere $C_{1-6}$-Alkylgruppen, $C_{1-6}$-Halogenoalkylgruppen oder Halogenatome subsituiert ist;
wobei jedoch der (3-Methoxybenzoylamino(hydroxy))-essigsäuremethylester ausgeschlossen ist,
bei welchem ein Amid der Formel $R^1CONH_2$, worin $R^1$ die oben angegebene Bedeutung besitzt, mit Methylglyoxylat in Reaktion gebracht wird.

2. Verfahren nach Anspruch 1, welches in einem flüssigen Kohlenwasserstofflösungensmittel und bei einer Temperatur im Bereich von 50 bis 120°C ausgeführt wird.

3. Verfahren nach Anspruch 2, bei welchem das Lösungsmittel aus Toluol und Xylolen ausgewählt wird.

4. Verfahren nach Anspruch 3, welches bei einer Temperatur über 100°C ausgeführt wird.

**Revendications pour les Etats contractants BE, CH, DE, FR, GB, IT, LI, NL, SE:**

1. Composés de formule générale (VI):

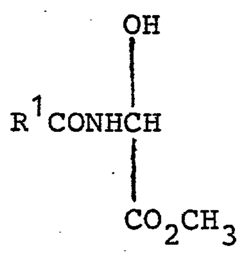

(VI)

dans laquelle $R^1$ représente:
(a) un groupe phényle facultativement substitué avec un ou plusieurs atomes d'halogènes, groupes cyano, groupes alkoxy en $C_1$ à $C_6$, groupes méthylène- ou éthylène-dioxy, groupes alkyle en $C_1$ à $C_6$ ou groupes halogénalkyle en $C_1$ à $C_6$ (par exemple des groupes trifluorométhyle); ou bien
(b) un groupe furyle, thiényle ou pyridyle facultativement substitué avec un ou plusieurs groupes alkyle en $C_1$ à $C_6$, groupes halogénalkyle en $C_1$ à $C_6$ ou atomes d'halogènes, mais à l'exclusion du (3-méthoxy-benzoylamino(hydroxy))acétate de méthyle.

2. Composés suivant la revendication 1, dans lesquels $R^1$ représente un groupe phényle facultativement substitué en l'une ou plusieurs des positions 3, 4 et 5 avec un ou plusieurs atomes d'halogènes ou groupes alkyle en $C_1$ à $C_6$, alkoxy en $C_1$ à $C_6$, trifluorométhyle ou méthylène- ou éthylène-dioxy.

3. Composés suivant la revendication 1 ou la revendication 2, dans lesquels $R^1$ représente un groupe phényle substitué en position 3 ou 4, ou bien en les deux positions, avec le chlore, le fluor, le brome, un groupe méthyle, méthoxy ou méthylènedioxy (3,4 seulement).

4. Composés de formule (VI) dans lequel:
$R^1$ représente un groupe 3,4-$OCH_2O.C_6H_3$, 4-Cl·$C_6H_4$, 4-$CH_3$·$C_6H_4$, 4-Br·$C_6H_4$, 4-$CH_3O$·$C_6H_4$, 3-F,4-$CH_3$·$C_6H_3$. ou 4-Cl-3,F-$C_6H_3$.

5. Composé suivant l'une quelconque des revendications précédentes, qui est sous forme d'un stéréoisomère.

6. Procédé de préparation d'un composé suivant la revendication 1, qui consiste à amener à réagir un amide de formule $R^1CONH_2$ dans laquelle $R^1$ répond à la définition suivant la revendication 1, avec le glyoxylate de méthyle.

7. Procédé suivant la revendication 6, mis en œuvre dans un solvant hydrocarboné liquide et à une température comprise dans l'intervalle de 50° à 120°C.

8. Procédé suivant la revendication 7, dans lequel le solvant est choisi entre le toluène et les xylènes.

9. Procédé suivant la revendication 8, mis en œuvre à une température supérieure à 100°C.

**Revendicatiobs pour l'Etat contractant AT:**

1. Procédé de préparation d'un composé de formule générale (VI):

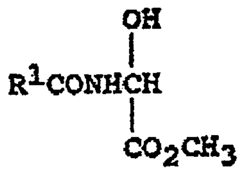

(I)

dans laquelle $R^1$ représente:

(a) un groupe phényle substitué avec un ou plusieurs atomes d'halogène, ledit groupe étant autre que le groupe 3,5-dichlorophényle, groupes cyano, groupes alkoxy en $C_1$ à $C_6$, groupes méthylène- ou éthylène-dioxy, groupes alkyle en $C_1$ à $C_6$ ou groupes halogénalkyle en $C_1$ à $C_6$ (par exemple des groupes trifluorométhyle); ou bien

(b) un groupe furyle, thiényle ou pyridyle facultativement substitué avec un ou plusieurs groupes alkyle en $C_1$ à $C_6$, groupes halogénalkyle en $C_1$ à $C_6$ ou atomes d'halogène, mais à l'exclusion du (3-méthoxy-benzoylamino(hydroxy))acétate de méthyle, qui consiste à amener à réagir un amide de formule $R^1CONH_2$, dans laquelle $R^1$ répond à la définition précitée, avec le glyoxylate de méthyle.

2. Procédé suivant la revendication 1, mis en œuvre dans un solvant hydrocarboné liquide et à une température comprise dans l'intervalle de 50°C à 120°C.

3. Procédé suivant la revendication 2, dans lequel le solvant est choisi entre le toluène et les xylènes.

4. Procédé suivant la revendication 3, mis en œuvre à une température supérieure à 100°C.